# EUROPÄISCHE PATENTSCHRIFT

(11) **EP 1 427 389 B1**
(45) Veröffentlichungstag und Bekanntmachung des Hinweises auf die Patenterteilung: **03.01.2007**
(21) Anmeldenummer: 02797633.1
(22) Anmeldetag: 28.08.2002
(51) Int. Cl.: A61K 8/44, A61Q 17/04, A61Q 19/00

(54) **ERHÖHUNG DER STABILITÄT VON KOSMETISCHEN ZUBEREITUNGEN DURCH ZUSATZ VON IMINODIBERNSTEINSÄURE**
INCREASE IN THE STABILITY OF COSMETIC PREPARATIONS CAUSED BY THE ADDITION OF IMINODISUCCINIC ACID
RENFORCEMENT DE LA STABILITE DE PREPARATIONS COSMETIQUES PAR ADJONCTION D'ACIDE IMINODISUCCINIQUE

(30) Priorität: 01.09.2001 DE 10142927
(43) Veröffentlichungstag der Anmeldung: 16.06.2004
(73) Patentinhaber: Beiersdorf AG, 20245 Hamburg (DE)
(72) Erfinder: KRÖPKE, Rainer, 22869 Schenefeld (DE); NIELSEN, Jens, 24558 Henstedt-Ulzburg (DE); GÖPPEL, Anja, 22527 Hamburg (DE)
(86) Internationale Anmeldenummer: PCT/EP2002/009576
(87) Internationale Veröffentlichungsnummer: WO 2003/020238

(56) Entgegenhaltungen:
- EP-A- 1 074 239
- WO-A-02/055050

## Beschreibung

Die vorliegende Erfindung betrifft die Verwendung von Iminodibernsteinsäure und/oder deren Salze zur Erhöhung der Stabilität von kosmetischen und/oder dermatologischen Zubereitungen,

Kosmetische und/oder dermatologische Zubereitungen die durchsichtig (transparent) bzw. durchscheinend (transluzent) sind erfreuen sich beim Verbraucher äußerster Beliebtheit. Da sie meistens durchsichtig sind, oftmals eingefärbt aber ebenso oft farblos klar sein dürften, bieten sie dem kosmetischen Entwickler zusätzliche Gestaltungsmöglichkeiten, die teilweise funktionalen Charakter haben, teilweise aber auch lediglich der Aufbesserung des äußeren Erscheinungsbildes dienen. So können beispielsweise dem Produkt, welches sich dem Betrachter dann in der Regel in einer durchsichtigen Verpackung darbietet, durch eingearbeitete Farbpigmente, Gasbläschen und dergleichen, oder aber auch größere Objekte, interessante optische Effekte verliehen werden.

Gerade dann, wenn es erwünscht ist, dass das oder die eingearbeiteten Objekte, mögen diese als solche mit dem bloßen Auge als solche erkenntlich sein, mögen sie in mikroskopischen Ausmaßen, aber in interessanter Anordnung - beispielsweise in Form von künstlich erzeugten Farbschlieren - dann doch sichtbare Formen ergeben, so ist es wünschenswert, dass diese Produkte in transparenten und/oder transluzenten Verpackungen aufbewahrt werden.

Auch für kosmetische und/oder dermatologische Zubereitungen die nicht transparent und/oder transluzent sind, werden transparente und/oder transluzente Verpackungen immer beliebter. Durchsichtige Glastiegel und Glasfläschchen gehören zu den am weit verbreitetsten Verpackungsformen für kosmetische und/oder dermatologische Zubereitungen. Sie wirken in ihren häufig kunstvoll verzierten Ausführungsformen als besonders edel und vermitteln dem Verbraucher den Eindruck mit dem Kosmetikum eine kleine Kostbarkeit und ein qualitativ hochwertiges Produkt erworben zu haben.

Leider sind jedoch kosmetische und/oder dermatologische Zubereitungen häufig nur begrenzt licht- und farbstabil, weshalb sie lichtgeschützt z.B. in Plastikflaschen aufbewahrt werden müssen.

Es war daher die Aufgabe der vorliegenden Erfindung, kosmetische und/oder dermatologische Zubereitungen so zu stabilisieren, dass sie in transparenten und/oder transluzenten Verpackungen problemlos aufbewahrt und den Verbrauchern zur Verfügung gestellt werden können.

Gelöst wurde die Aufgabe überraschender Weise durch die Verwendung von lminodibernsteinsäure und/oder deren Salzen zur Erhöhung der Farb- und Lichtstabilität kosmetischer und/oder dermatologischer Zubereitungen.

Erfindungsgemäß vorteilhaft ist dabei der Einsatz von Iminodibernsteinsäure und/oder deren Salzen in einer Konzentration von 0,001 bis 15 Gewichts-% bezogen auf das Gesamtgewicht der Zubereitung.

Es ist hierbei erfindungsgemäß vorteilhaft, das Tetranatriumsalz als bevorzugte Iminodibernsteinsäureverbindung einzusetzen.

Iminodibernsteinsäure hat folgende Struktur, wobei vermutlich ein Gleichgewicht tautomerer Formen vorliegt:

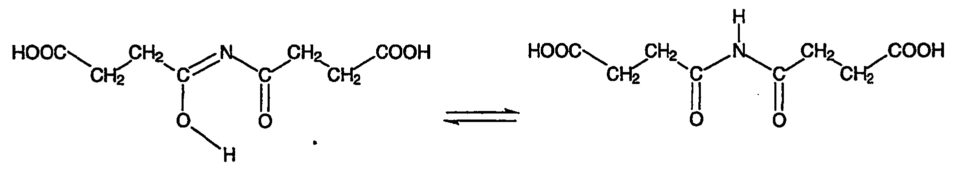

Iminodibersteinsäure ist u.a. von der Fa. Bayer AG unter den Handelsnamen Iminodisuccinat VP OC 370 (ca. 30% ige Lösung) und Baypure CX 100 fest erhältlich.

Die Verwendung von Iminodibernsteinsäure und/oder deren Salzen führt zu einer Erhöhung der Farb-, Licht- und Geruchsstabilität kosmetisch und/oder dermatologischer Zubereitungen. Insbesondere wird die Farb-, Licht- und Geruchsstabilität kosmetisch und/oder dermatologischer Zubereitungen in transparenten und/oder transluzenten Verpackungen durch die. Zugabe von Iminodibernsteinsäure und/oder deren Salze erhöht.

Vorteilhaft sind Produkte bestehend aus einer kosmetischen und/oder dermatologischen Zubereitung enthaltend Iminodibernsteinsäure und/oder deren Salze und einer transparenten und/oder translucenten Verpackung.

Diese kosmetischen und/oder dermatologischen Produkte können vorteilhaft verwendet werden als Hautpflegeprodukte, als Gesichtspflegeprodukte sowie als Sonnenschutzprodukte. Dabei versteht man unter "Hautpflegeprodukte" im Sinne der Erfindung unter anderem Hautcremes, Hautlotionen, Milche, Salben, Öle, Balsame und Sera die der Pflege der Haut dienen. Gesichtspflegeprodukte dienen als eine Sonderform der Hautpflegeprodukte der Pflege der Gesichtshaut. Insbesondere dienen sie der Verhinderung entstehender und/oder Verminderung bereits bestehender Fältchen und Falten. Gesichtspflegeprodukte beinhalten erfindungsgemäß auch dekorative Kosmetika, deren Hauptzweck die Farbänderung von Haut und Hautanhangsgebilden (z.B. Wimpern, Augenbrauen) ist. Unter Sonnenschutzprodukten im Sinne der Erfindung sind alle Formen von Zubereitungen zu verstehen, welche UV-Lichtschutzfilter enthalten. Des Weiteren beinhalten sie sogenannte "Aftersun-Produkte". Diese sind dazu bestimmt, die Haut nach dem Sonnenbad zu kühlen und ihr Feuchthaltevermögen zu verbessern, wobei die Vermittlung des Kühleffektes eine zentrale Rolle spielt. Dieser Kühleffekt wird in der Regel durch hohe Mengen an Ethanol und Wasser erzielt, welches beim Verteilen der Formulierung auf der Haut spontan verdunstet. Femer enthalten diese Präparate meist Feuchthaltemittel wie Glycerin oder Propylenglycol und entzündungshemmende Verbindungen wie zum Beispiel Allantion, α-Bisabolol, Panthenol oder Aloe-vera-Extrakt.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **Beispiel Nummer** | **1** | **2** | **3** | **4** | **5** | **6** | **7** | **8** |
|---|---|---|---|---|---|---|---|---|
| Glycerylstearatcitrat | 2 | | | | | | | |
| Glycerylsterat | | 5 | 2 | 3 | | | | |
| PEG-40-Stearat | | | 1 | | | | | |
| PEG-100-Stearat | | | | 1 | | | | |
| Triglycerinmethylgluco-sedistearat | | | | | 3 | | | |
| Sorbitanstearat | | | | | 1 | | | |
| Polyethylenglycol(21)stearyl-ether | | | | | | 2 | | |
| Polyethylenglycol(2)stearyl-ether (Steareth-2) | | | | | | 1 | | |
| Cetearylglucosid | | | | | | | 2 | |
| Stearinsäure | | | | | | | | 2,5 |
| Myristylmyristat | 1 | | 1 | | | | 1 | |
| Behenylalkohol | | | | | 1 | | | 2 |
| Stearylalcohol | 2 | 1 | | | | | 5 | |
| Cetearylalkohol | | | 2 | | | 2 | | 1 |
| Cetylalcohol | 1 | | | 1 | 1 | | | |
| Hydrierte Cocosfettglyceride | 2 | | | 1 | | | | 1 |
| Sheabutter | | 2 | | | | | | |
| C12-15 Alkylbenzoat | | 3 | 3 | | 2 | 5 | 2 | |
| Butylenglycol Dicaprylat/Dicaprat | 1 | | | | | | | 2 |
| Caprylic/Capric Triglyceride | | 4 | | | 1 | | | |
| Hydriertes Polydecen | | | | | | | 1 | |
| Ethylhexylkokosfettsäureester | 3 | | | | | | | 2 |
| Octyldodecanol | | | | | | 1 | | |
| Mineralöl | | 1 | | | | 1 | | |
| Vaseline | 4 | | | 2 | | | | |
| Octamethyltetrasiloxan | | 1 | 3 | 1 | 3 | 2 | | |
| Dimethylpolysiloxan | | | 1 | | | | | 1 |
| Dicaprylylether | 1 | 4 | | | | | | |
| Dicarprylylcarbonat | | | | | 2 | | | 4 |
| Polydecen | | | | 1 | | | 5 | |
| TiO₂ | | | 1 | 1 | | | 2 | 1 |
| Ethylhexylmethoxycinnamat | 3 | 2 | | 5 | 3 | | | 3 |
| 2-Ethylhexyl-2-cyano-3- diphenylacrylat | | | 5 | | | | | |
| Ethylhexyltriazon | | 2 | | | 2 | 3 | | |
| Butylmethoxydibenzoyl-methan | | 1 | | 1 | | | | |
| Bis-Ethylhexyloxyphenol- methoxyphenyltriazine | 1 | | | | 1 | | | 2 |
| Ubichinon (Q10) | 0,05 | | 0.05 | | 0,02 | | | 0,1 |
| Retinylpalmitat | | | | | | | 0,1 | |
| Tocopherylacetat | | 0.5 | | | | | | |
| α-Glucosylrutin | | | | | | 0.1 | | |
| Ascorbinsäure | | | | 0,2 | | | | |
| Liponsäure | | | 0,1 | | | | | |
| Retinol | | | | | 0.1 | | | |
| Iminodibernsteinsäure | | 0,2 | | | | | | |
| Tetranatriumiminodisuccinat | 0,1 | --- | 0,3 | 0,1 | 0,1 | 0.2 | 0,5 | 0,25 |

| **Beispiel Nummer** | **1** | **2** | **3** | **4** | **5** | **6** | **7** | **8** |
|---|---|---|---|---|---|---|---|---|
| Phenoxyethanol | 0,3 | | | 0,8 | 0,4 | 0,5 | | 0,3 |
| p-Hydroxybenzoesäureal-kylester | 0,5 | 0,4 | 0,3 | 0,4 | | | 0,4 | 0,6 |
| Hexamidindiisethionat | | | 0,04 | 0,05 | | 0,1 | | |
| Diazolidinylhamstoff | 0,25 | | | | 0,2 | | 0,1 | |
| 1,3-Dimethylol-5,5-dimethyl-hydantoin | | 0,2 | | | | | | |
| lodopropynylbutylcarbamat | 0,1 | | 0.05 | | | 0,20 | | |
| Ethanol denaturiert | 1 | 2 | | | 8 | | | 3 |
| 2-Ethylhexylglycerinether | | | | 3 | | | | |
| Xanthan Gummi | 0,1 | | | 0,2 | 0,1 | 0,1 | | |
| Polyacrylsäure - | 0,2 | | 0,1 | | | 0,2 | | |
| Polyacrylamid | | 0,2 | | | 0,2 | | | |
| Glycerin | 8 | 10 | 5 | 15 | 5 | 6 | 4 | 5 |
| Butylenglycol | | 1 | 2 | | | | 2 | |
| Wasser- und/oder öllösliche Farbstoffe | 0,05 | | | | | | | 0,1 |
| Füllstoffe (Distärkephosphat, SiO₂, Talkum, Aluminiumstearat) | | | | | | | 5 | 1 |
| Parfüm | q.s. | q.s. | q.s. | q.s. | q.s. | q.s. | q.s. | q.s. |
| Wasser | Ad 100 | Ad 100 | Ad 100 | Ad 100 | Ad 100 | Ad 100 | Ad 100 | Ad 100 |

## Patentansprüche

1. Verwendung von Iminodibernsteinsäure und/oder deren Salze zur Erhöhung der Farb- und Lichtstabilität kosmetischer und/oder dermatologischer Zubereitungen.

2. Verwendung von Iminodibernsteinsäure und/oder deren Salze zur Erhöhung der Farb- und Lichtstabilität kosmetischer und/oder dermatologischer Zubereitungen in transparenten und/oder translucenten Verpackungen.

## Claims

1. Use of iminodisuccinic acid and/or salts thereof to increase the color- and light-stability of cosmetic and/or dermatological formulations.

2. Use of iminodisuccinic acid and/or salts thereof to increase the color- and light-stability of cosmetic and/or dermatological formulations in transparent and/or translucent packaging.

## Revendications

1. Utilisation de l'acide iminodisuccinique et/ou de ses sels pour renforcer la stabilité de la couleur et la stabilité à la lumière de préparations cosmétiques et/ou dermatologiques.

2. Utilisation de l'acide iminodisuccinique et/ou de ses sels pour renforcer la stabilité de la couleur et la stabilité à la lumière de préparations cosmétiques et/ou dermatologiques dans des emballages transparents et/ou translucides.
